# EUROPÄISCHE PATENTSCHRIFT

(11) **EP 4 058 415 B1**
(45) Veröffentlichungstag und Bekanntmachung des Hinweises auf die Patenterteilung: **31.07.2024**
(21) Anmeldenummer: 20804250.7
(22) Anmeldetag: 10.11.2020
(51) Int. Cl.: C03C 15/00, C03C 23/00, C12M 1/32

(54) **EINTEILIGE REAKTIONSGEFÄSSE AUS GLAS, HERSTELLUNGSVERFAHREN UND VERFAHREN ZU ANALYSE**
SINGLE-PIECE REACTION VESSEL MADE OF GLASS, PRODUCTION METHOD, AND ANALYSIS METHOD
RÉCIPIENT DE RÉACTION MONOBLOC EN VERRE, PROCÉDÉ DE PRODUCTION ET PROCÉDÉ D'ANALYSE

(30) Priorität: 12.11.2019 DE 102019217466
(43) Veröffentlichungstag der Anmeldung: 21.09.2022
(73) Patentinhaber: LPKF Laser & Electronics SE, 30827 Garbsen (DE)
(72) Erfinder: KRÜGER, Robin, 30419 Hannover (DE); SCHULZ-RUHTENBERG, Malte, 31515 Wunstorf (DE); VAN AALST, Jan, 30827 Garbsen (DE); WOLLER, Moritz, 31535 Neustadt (DE)
(74) Vertreter: Taruttis, Stefan Georg
(86) Internationale Anmeldenummer: PCT/EP2020/081585
(87) Internationale Veröffentlichungsnummer: WO 2021/094286

(56) Entgegenhaltungen:
- US-A- 5 919 607
- US-A1- 2009 013 724
- US-A1- 2009 261 082
- US-A1- 2010 050 692
- US-A1- 2013 029 875

## Beschreibung

Die vorliegende Erfindung betrifft ein Verfahren zur Herstellung von Reaktionsgefäßen aus Glas, die in Form von Ausnehmungen in Glas geformt sind. Das Glas, in dem die Reaktionsgefäße in einer Anordnung eingeformt sind, ist einstückig, so dass die Reaktionsgefäße in einteiligem Glas, insbesondere einer Glasplatte, ausgebildet sind. Das Verfahren erzeugt eine Anordnung einer Vielzahl von Reaktionsgefäßen, die in einstückigem Glas eingeformt ist, z.B. eine Anordnung von 25 x 25 Feldern von jeweils 8 x 12 Reaktionsgefäßen. Die Reaktionsgefäße sind in einer Vielzahl in einer einstückigen Glasplatte ausgebildet, die aus einer ersten Glasplatte besteht.

Das Verfahren hat den Vorteil, ohne mechanische Einwirkung auf ein massives Glas Ausnehmungen zu formen, die Reaktionsgefäße bilden, die daher keine mechanischen Beschädigungen aufweisen, z.B. keine Mikrorisse. Die Reaktionsgefäße weisen ein großes Streckungsverhältnis von Tiefe zu Durchmesser auf. Ein weiterer Vorteil liegt darin, dass das Verfahren zur Herstellung der Reaktionsgefäße zumindest ohne selektive Beschichtung der Glasoberfläche ablaufen kann, in der die Querschnittsöffnungen der Reaktionsgefäße gebildet sind, optional ohne jede Beschichtung der Oberflächen des Glases, in dem die Reaktionsgefäße gebildet werden.

### Stand der Technik

Deutsch et al., Lab Chip, 2006, 69, 995-1000, beschreiben die Herstellung von Reaktionsgefäßen eines Durchmessers von 20 µm bei 8 µm Tiefe in einer Glasplatte durch Ätzen nach dem Auftragen einer Maske aus Chrom und darauf Photoresist.

Die US 2003/0211014 A1 beschreibt die Herstellung von Reaktionsgefäßen in Glas mittels eines mit Ultraschall beaufschlagten Werkzeugs und Schleifmittel zwischen dem Werkzeug und dem Glas.

Die EP 1 867 612 A1 beschreibt Mikrotiterplatten mit 96 Näpfen aus einem Boden aus Glas, das für UV durchlässig ist und durch Glasfritte mit einer Glasplatte verbunden ist, in der durchgehende Ausnehmungen die Seitenwände der Näpfe bilden.

Die EP 2 011 857 A1 beschreibt das Erzeugen von Oberflächenstrukturen am Boden von Mikrotiterplatten mittels eines photolithographischen Verfahrens.

Die US 2010/0050692 A1 beschreibt das Einstrahlen von Laserstrahlung einer Wellenlänge auf Glas, das eine optische Dichte von zumindest 1,5 für diese Wellenlänge hat, diese Wellenlänge absorbiert und sich durch das Einstrahlen erwärmt und dadurch lokal schwillt. Anschließend wird das Glas geätzt.

Die US 5 919 607 A beschreibt das Bestrahlen von Glas mit frequenzgedoppeltem Laserlicht von 532 nm und Erzeugen von Gräben in der Glasoberfläche durch anschließendes Ätzen, optional anschließendem Polieren.

Die US 2009/0013724 A1 beschreibt das Einstrahlen von Laserpulsen von 535 nm oder darunter mit einer Pulsdauer von 1 ns bis 200 ns durch eine Linse, die die Laserpulse innerhalb der Glasdicke fokussiert. Anschließendes Ätzen erzeugt Sacklöcher oder Durchgangslöcher im Glas.

Die US 2013/00298875 A1 beschreibt in den Kunststoff PDMS als Bodenplatte eines Napfs eingelassene Glasplatte mit Durchgangslöchern zur Zellkultivierung.

Die US 2009/0261082 A1 beschreibt eine Rastereinrichtung zur Führung von zwei gepulsten Laserstrahlen über eine Glasplatte, aus der durch die Laserbestrahlung Glas abgetragen wird.

### Aufgabe der Erfindung

Der Erfindung stellt sich die Aufgabe, ein alternatives Herstellungsverfahren bereitzustellen, das insbesondere geeignet ist, Reaktionsgefäße mit einem großen Streckungsverhältnis bei insgesamt kleinem Volumen in Glas herzustellen, sowie eine Anordnung einer Vielzahl solcher Reaktionsgefäße in Glas bereitzustellen. Bevorzugt soll das Verfahren geeignet sein, eine Anordnung solcher Reaktionsgefäße bereitzustellen, bei der das Glas bei Bestrahlung mit Licht einen hohen optischen Kontrast zu Zellen in den Reaktionsgefäßen bildet.

### Beschreibung der Erfindung

Die Erfindung löst die Aufgabe mit den Merkmalen der Ansprüche und insbesondere mit einem Verfahren zur Herstellung von Reaktionsgefäßen aus Glas sowie den mit dem Verfahren erhältlichen Reaktionsgefäßen aus Glas. Das Verfahren weist die Schritte
1. Einstrahlen, bevorzugt punktförmiges und weiter bevorzugt senkrechtes Einstrahlen eines Laserstrahls einer Wellenlänge, für die eine Glasplatte durchlässig ist, auf die Stellen der Oberfläche der Glasplatte, an denen jeweils eine Ausnehmung als
2. Reaktionsgefäß erzeugt werden soll, wobei der Laserstrahl aus Laserpulsen mit Pulslängen von maximal 100 ps besteht, und die Fokuslage der Laserpulse so eingestellt wird, dass sich deren Fokus, insbesondere in Richtung der Ausbreitungsrichtung der Laserstrahlung, nicht über die gesamte Dicke der Glasplatte erstreckt und dass die erste Glasplatte an den Stellen, an denen eine Ausnehmung erzeugt werden soll, an mehreren voneinander beabstandeten Positionen mit Laserpulsen bestrahlt wird,
3. Ätzen der Glasplatte, bevorzugt für eine Zeitdauer, die zum Erzeugen von Ausnehmungen einer Tiefe, bevorzugt von zumindest 40 µm oder zumindest 30 µm, bevorzugt mit einem Streckungsverhältnis von zumindest 2, zumindest 4, zumindest 5 oder zumindest 6 von Tiefe zu Durchmesser, der in der Ebene der ersten Oberfläche gemessen ist, entlang der Stellen ausreicht, um die Ausnehmungen zu erzeugen,
wobei das Ätzen der Glasplatte beendet wird, wenn sich die Ausnehmungen nur über einen Anteil der Dicke der ersten Glasplatte erstrecken und daher die Ausnehmungen einen einstückig in der ersten Glasplatte ausgebildeten Boden aufweisen, so dass bevorzugt die Böden der Ausnehmungen, die die Reaktionsgefäße bilden, an jeder Position, auf die ein Laserpuls eingestrahlt wurde, eine Ausnehmung aufweist oder an jeder Position, auf die ein Laserpuls eingestrahlt wurde, von Ausnehmungen gebildet sind, oder besteht daraus.

Das Einstellen der Fokuslage der Laserpulse so, dass sich deren Fokus, insbesondere in Richtung der Ausbreitungsrichtung der Laserstrahlung, nicht über die gesamte Dicke der Glasplatte erstreckt, wird dadurch erreicht, dass die Fokuslage der Laserpulse so eingestellt wird, dass sich deren Fokus, insbesondere in Richtung der Ausbreitungsrichtung der Laserstrahlung, nur über einen Anteil Dicke der Glasplatte erstreckt, z.B. über einen ersten Dickenabschnitt der Glasplatte.

Jede Glasplatte kann hierbei ein Gegenstand aus Glas mit einer lateralen Ausdehnung sein, die größer ist als die Dicke. Glasplatten können also einen rechteckigen, runden oder anders geformten Umfang aufweisen, der ihre gegenüberliegenden Oberflächen begrenzt.

Die Reaktionsgefäße werden durch Ausnehmungen in einer Glasplatte gebildet, wobei die Ausnehmungen genau eine Öffnung aufweisen, die in der Ebene einer ersten Oberfläche der Glasplatte liegt. Anwendungen der Reaktionsgefäße sind nicht auf chemische Reaktionen beschränkt, sondern schließen biochemische, biologische und physikalische Prozesse ein.

Dies können Prozesse mit einzelnen Zellen, die tierische oder pflanzliche Zellen oder Hefezellen sein können, Bakterien, Viren, Proteinen etc. oder mit Clustern dieser sein.

Das punktförmige Einstrahlen wird durch Fokussieren der Laserstrahlung auf einen Punkt mit einer Größe von wenigen Mikrometern, z.B. 1 bis 10 µm oder bis 5 µm, erzielt. Dabei ist es vorteilhaft, wenn der Fokus der Laserstrahlung sich über eine Länge entlang der Ausbreitungsrichtung des Laserstrahls erstreckt, die wesentlich größer ist als die Rayleigh-Länge eines entsprechenden Laserstrahls mit Gauß-Profil. Dies kann durch geeignete optische Einrichtungen, z.B. diffraktiv-optische Elemente, erreicht werden. Das Einstrahlen ist ein Einstrahlen, das die Glasplatte nicht durchstrahlt, sondern weniger tief bis in einen an die erste Oberfläche angrenzenden ersten Dickenabschnitt der ersten Glasplatte erfolgt, dadurch dass sich der Fokus der Laserstrahlung in Richtung der Ausbreitungsrichtung der Laserstrahlung nicht über die gesamte Dicke der Glasplatte erstreckt, sondern sich die Laserstrahlung von einer ersten Oberfläche der Glasplatte nur bis in einen an die erste Oberfläche angrenzenden ersten Dickenabschnitt erstreckt und im ersten Dickenabschnitt endet, so dass sich die Laserstrahlung nicht bis in einen daran anschließenden zweiten Dickenabschnitt der Glasplatte erstreckt. Da eine Wechselwirkung zwischen Laserstrahlung und Material der Glasplatte nur im Fokus stattfindet, ist es so möglich, den Wechselwirkungsbereich innerhalb eines ersten Dickenabschnitts der Glasplatte enden zu lassen. Dabei besteht die Laserstrahlung aus Laserpulsen.

Die erste Oberfläche der ersten Glasplatte, sowie in Abwesenheit einer Beschichtung aus Ätzresist auch die dieser gegenüberliegende zweite Oberfläche, wird beim Ätzen an den Stellen, auf die der Laser auf die Glasplatte eingestrahlt wurde und an denen der Laserstrahl gegenüber ausgetreten ist, deutlich schneller abgetragen, als die benachbarten Bereiche. Die Bereiche der ersten Oberfläche der Glasplatte werden daher wegen der Abwesenheit einer Beschichtung, z.B. aus Ätzresist, im Abstand von den Stellen der punktförmigen Laserbestrahlung langsamer und gleichförmig abgetragen. Daher wird die erste Oberfläche mit Ausnahme der Ausnehmungen von Oberflächenabschnitten gebildet, die in einer Ebene angeordnet sind, aus der sich die Ausnehmungen in das Glasvolumen der Glasplatte erstrecken. Die Oberflächenabschnitte, die in einer gemeinsamen Ebene angeordnet sind und die erste Oberfläche bilden, von der die Ausnehmungen ausgenommen sind, werden von den Stirnflächen der Wandungen gebildet, die zwischen den Ausnehmungen liegen.

Generell optional kann bei einem Verfahren mit oder aus den Schritten die zweite Oberfläche der Glasplatte durchgängig mit Ätzresist beschichtet sein, um die Ausbildung von Ausnehmungen von der zweiten Oberfläche in die Glasplatte bzw. das gleichmäßige Abtragen von dieser Oberfläche aus zu verhindern. Erfindungsgemäß ist der pulsförmige Laserstrahl beim Einstrahlen gesteuert, dass die Intensität nur ausreicht, um einen ersten Anteil der Dicke der ersten Glasplatte zu durchqueren, um die Glasplatte entlang des Lichtpfads zu modifizieren, z.B. dadurch, dass der Abstand der Fokuslage relativ zur ersten Oberfläche der Glasplatte eingestellt wird. Dazu ist die Fokuslage der Laserpulse so eingestellt, dass die Laserpulse nur bis in einen Dickenabschnitt in die erste Glasplatte eindringen.

Optional sind die in Schritt 1 punktförmig eingestrahlten Laserstrahlen, an denen in Schritt 2 eine Ausnehmung, die ein Reaktionsgefäß bilden soll, geätzt wird, in der Ebene der ersten Oberfläche der Glasplatte in einem Abstand von zumindest oder genau dem Durchmesser eines der Reaktionsgefäße zuzüglich der Dicke einer Wand zwischen den Reaktionsgefäßen angeordnet. Der Durchmesser der Reaktionsgefäße ist durch die Reaktionsbedingungen und die Dauer des Ätzens einstellbar, da das Ätzen konzentrisch um den linearen Pfad erfolgt, den der eingestrahlte Laserstrahl durch die Glasplatte genommen hat. Die Wände, die zwischen den Reaktionsgefäßen angeordnet sind, enden in einer gemeinsamen Ebene. Die Endflächen dieser Wände liegen in einer gemeinsamen Ebene und bilden die erste Oberfläche bzw. bilden in einer gemeinsamen Ebene die erste Oberfläche, wobei die erste Oberfläche von den Ausnehmungen unterbrochen ist. Die erste Oberfläche wird bevorzugt nur von den Querschnitten der Ausnehmungen unterbrochen.

Der Laserstrahl ist bevorzugt an jeder der Stellen, an denen er auf die Glasplatte eingestrahlt wird, gepulst, z.B. mit einer Wellenlänge von 1064 nm bis 515 nm, mit Pulslängen von maximal 100 ps oder maximal 50 ps, bevorzugt maximal 10 ps. Generell ist der Laser eingerichtet, dass der Laserstrahl zwischen den Stellen nicht auf die Glasplatte trifft. Bevorzugt wird der Laserstrahl punktförmig und senkrecht auf die Oberfläche der Glasplatte eingestrahlt. Bevorzugt bildet diese Oberfläche, auf die der erste Laserstrahl eingestrahlt wurde, die erste Oberfläche der ersten Glasplatte.

Generell wird die Glasplatte auch als erste Glasplatte bezeichnet.

Das Ätzen erfolgt z.B. mit Flusssäure, z.B. 1 bis 48 Gew.-%, und/oder Schwefelsäure und/oder Salzsäure und/oder Phosphorsäure und/oder Salpetersäure, oder Kalilauge, bei z.B. bis zu 140°C.

Die Glasplatte kann z.B. eine Dicke vor dem Ätzen von bis zu 1000 µm, bevorzugt 100 bis 1000 µm, z.B. bis 800 µm, z.B. 300 bis 500 µm aufweisen, nach dem Ätzen eine um 50 bis 700 µm geringere Dicke, z.B. eine bis 200 µm geringere Dicke.

In Schritt 3 wird das Ätzen der Glasplatte beendet, wenn sich die Ausnehmungen nur über einen Anteil der Dicke der Glasplatte erstrecken, so dass die Tiefe der Ausnehmungen in die erste Glasplatte nur einen Anteil der Dicke der Glasplatte beträgt und daher die Ausnehmungen einen einstückig in der Glasplatte ausgebildeten Boden aufweisen.

Die Ausnehmungen erstrecken sich bevorzugt in einem Winkel von z.B. 0° bis 15° kegeloder kegelstumpfförmig zulaufend und von der ersten Oberfläche der Glasplatte ausgehend in deren Volumen.

Optional kann generell die Glasplatte ohne eine Beschichtung, z.B. ohne Maske und/oder ohne Ätzresist, dem Ätzen unterzogen werden, so dass das Verfahren den Vorteil hat, ohne Auftragen und ohne Entfernen von Ätzresist von einer Glasplatte durchgeführt zu werden. Generell bleibt zumindest die erste Oberfläche der Glasplatte ohne Ätzresist und ohne Maske und wird ohne Ätzresist geätzt.

Generell optional, insbesondere bei einem Verfahren mit oder aus den Schritten 1 bis 3, wird die zweite Oberfläche der Glasplatte vollständig mit Ätzresist beschichtet, so dass das Ätzen nur von der ersten Oberfläche her erfolgt, oder der Laser, der den Laserstrahl einstrahlt, ist eingerichtet, dass der Laserstrahl nur einen Anteil der Dicke der Glasplatte durchquert, bzw. dass der Laserstrahl innerhalb der Dicke der Glasplatte endet. In dieser Ausführungsform wird jede Stelle der Glasplatte, an denen eine Ausnehmung erzeugt werden soll, an mehreren voneinander beabstandeten Positionen, z.B. an zumindest 2 oder zumindest 3 oder zumindest 10 oder zumindest 30 Positionen, mit Laserstrahlen punktförmig bestrahlt, wobei die Laserstrahlen bevorzugt parallel zueinander und senkrecht auf die Glasplatte eingestrahlt werden, nacheinander oder gleichzeitig. Die Positionen bilden die Stelle, an der das Ätzen die Glasplatte schneller abträgt, als an davon entfernten Oberflächenbereichen. Die Positionen, an denen der Laser eingestrahlt wurde, führen beim Ätzen zu einem gleichmäßig schnellen Abtrag des Glases und bilden gemeinsam eine Ausnehmung. Die Positionen, die im Bereich einer Stelle eingestrahlt werden und eine Stelle bilden, sind z.B. in einem Abstand von 1 bis 10 µm angeordnet. Bevorzugt sind die Positionen innerhalb des Bereichs um jede Stelle angeordnet, in dem jeweils eine Ausnehmung gebildet werden soll. Bevorzugt sind die Positionen, an denen Laserstrahlen um eine Stelle oder zur Ausbildung einer Stelle eingestrahlt werden, in einem Abstand von 1 bis 10 µm, z.B. 2 bis 5 µm oder bis 3 µm, der insbesondere in der Ebene der ersten Oberfläche der ersten Glasplatte bestimmt ist.

Generell kann eine Ausnehmung durch einen einzelnen Laserpuls oder mehrere Laserpulse erzeugt werden. Bei einem einzelnen Laserpuls wird der Durchmesser der Ausnehmung vornehmlich durch die Ätzdauer bestimmt. Bei der Erzeugung einer Ausnehmung mit mehreren Laserpulsen wird der Durchmesser der Ausnehmung von Anzahl und Abstand der Positionen bestimmt, an denen Laserstrahlen für eine Stelle eingestrahlt werden und in die erste Glasplatte eindringen. Die Tiefe der Ausnehmung in das Volumen der ersten Glasplatte kann durch die Zeitdauer des Ätzens bestimmt werden und dadurch, dass der Laserstrahl nur zu einem Anteil der Dicke in die Glasplatte eindringt und die Glasplatte nicht vollständig durchstrahlt.

Optional wird um jede Stelle, an der eine Ausnehmung gebildet werden soll, auf einem umfänglich geschlossenen Weg, der bevorzugt ringförmig, rechteckig oder hexagonal ist, ein Laserstrahl eingestrahlt, der z.B. durch nebeneinander eingestrahlte Laserstrahlpulse gebildet wird. Dabei können Laserstrahlpulse auf dem umfänglich geschlossenen Weg auf die erste Glasoberfläche eingestrahlt werden, z.B. in einem auf der ersten Oberfläche der ersten Glasplatte bestimmten Abstand der Laserstrahlpulse von 3 µm nebeneinander. Optional kann daher die Glasplatte an jeder Stelle an mehreren voneinander beabstandeten Positionen mit Laserstrahlen jeweils punktförmig bestrahlt werden und um diese Positionen herum ein Laserstrahl, z.B. durch nebeneinander eingestrahlte Laserstrahlpulse gebildet, entlang eines umfänglich geschlossenen Wegs eingestrahlt werden. Das Einstrahlen eines Laserstrahls entlang eines umfänglich geschlossenen Wegs hat den Vorteil, dass beim anschließenden Ätzen Ausnehmungen mit einer Wandung gebildet werden, die sich von der ersten Oberfläche erstreckt und einen Querschnitt aufweist, der den umfänglich geschlossenen Weg einschließt.

Daher kann die Glasplatte einstückig die Reaktionsgefäße als Ausnehmungen aufweisen. In dieser Ausführungsform wird der Boden der Reaktionsgefäße vom Material der Glasplatte gebildet. Dabei ist bevorzugt der Boden der Reaktionsgefäße von einer Vielzahl aneinander angrenzender Ausnehmungen gebildet, die nebeneinander etwa in einer Ebene angeordnet sind, die parallel zur ersten Oberfläche und parallel zur Ebene der zweiten Oberfläche der Glasplatte liegt.

In dieser Ausführungsform kann der Boden der Ausnehmungen Mikrostrukturen aufweisen, die für eine Nahfeldbeleuchtung des Innenvolumens der Ausnehmungen eingerichtet sind. Solche Mikrostrukturen können z.B. die Form von schmalen, hohen Glasspitzen haben und so als Lichtwellenleiter für die Beleuchtung fungieren oder einzelne Zellen bzw. Ansammlungen von Zellen in ihrer Lage oder Position beeinflussen können. Solche Strukturen können hergestellt werden, indem z.B. eine Ausnehmung durch das Aufätzen einer Vielzahl von Positionen, an denen eng beabstandet Laserpulse eingestrahlt wurden, gebildet wird. Wird in der Mitte der Ausnehmung ein einzelner Laserpuls ausgelassen, so bleibt nach dem Ätzprozess hier eine Glasspitze innerhalb der Ausnehmung stehen. Generell kann im Verfahren eine Ausnehmung durch Einstrahlen von Laserpulsen nebeneinander an Positionen und anschließendes Ätzen erzeugt werden, wobei die Positionen in jeweils gleichen Abständen voneinander von maximal 10 µm, z.B. 1 bis 5 µm oder bis 3 µm angeordnet sind und zusammen eine Stelle bilden, wobei zumindest 2 oder zumindest 3 Positionen in einem größeren Abstand angeordnet sind, z.B. in einem Abstand von 10 bis 30 µm, z.B. 15 bis 20 µm Abstand. Die zumindest 2 oder 3 Positionen der Laserpulse, die in einem größeren Abstand angeordnet sind, weisen zwischen sich z.B. den Bereich auf, in dem ein Laserpuls im gleichen Abstand ausgelassen ist, bzw. umgeben den Bereich, in dem beim Ätzen eine Glasspitze stehenbleibt.

Generell können Laserpulse an Positionen, die eine Stelle bilden, an der durch Ätzen eine Ausnehmung gebildet wird, bis in unterschiedliche Tiefen in die Glasplatte eingestrahlt werden. So können z.B. Laserpulse an Positionen tiefer in die Dicke der Glasplatte eingestrahlt werden und tiefer eindringen und andere Laserpulse an Positionen weniger tief in die Dicke der Glasplatte eingestrahlt werden. Beim anschließenden Ätzen werden an den Positionen, an denen Laserpulse tiefer in die Glasplatte eingestrahlt wurden, tiefere bzw. weitere Ausnehmungen gebildet, und an den Positionen, an denen Laserpulse weniger tief in die Glasplatte eingestrahlt wurden, wird der Boden der Ausnehmung in geringerer Tiefe gebildet. Generell kann, abhängig vom Abstand der Positionen, an jeder Position eine konkave Vertiefung im Boden gebildet werden. Eine Ausnehmung, die einen Boden und darin weitere tiefere Ausnehmungen aufweist, kann durch Einstrahlen von Laserpulsen in dem Teil der Positionen, die den Boden bilden sollen, bis weniger tief in die Glasplatte und Einstrahlen von Laserpulsen in dem Teil der Positionen, die weitere Ausnehmungen, die sich ausgehend von dem Boden tiefer in die Glasplatte erstrecken sollen, bis tiefer in die Glasplatte und anschließendes Ätzen hergestellt werden. Für einen größeren Durchmesser weiterer Ausnehmungen, die sich ausgehend vom Boden einer Ausnehmung tiefer in die Glasplatte erstrecken, können Laserpulse, die tiefer in die Glasplatte eingestrahlt werden, an benachbarten Positionen, z.B. in einem Abstand von 1 bis 10 µm, z.B. 2 bis 5 oder bis 3 µm angeordnet sein, so dass an diesen Positionen das Ätzen tiefer in die Glasplatte eindringt. So können an einem Teil der Positionen Laserpulse weniger tief in die Glasplatte eingestrahlt werden, und an einem Teil der Positionen Laserpulse tiefer in die Glasplatte eingestrahlt werden, so dass beim Ätzen an den Positionen, an denen die Laserpulse weniger tief eingestrahlt wurden, ein Boden mit konkaven Vertiefungen in geringerer Tiefe erzeugt wird und an den Positionen, an denen Laserpulse tiefer eingestrahlt wurden, weitere Ausnehmungen erzeugt werden, die sich tiefer in die Glasplatte erstrecken.

In dieser Ausführungsform zur Herstellung von Reaktionsgefäßen, die in einer einstückigen Glasplatte, bzw. von nur einer Glasplatte ausgebildet sind, wird das Ätzen für eine Zeitdauer durchgeführt, die zum Erreichen einer gewünschten Tiefe der Ausnehmungen im Glasvolumen der Glasplatte ausreicht, die in einem Abstand von der zweiten Oberfläche liegt, bzw. nur für eine Zeitdauer geätzt wird, nach der die Glasplatte noch eine geschlossene zweite Oberfläche aufweist. Optional kann dabei der Boden der Reaktionsgefäße konkave Vertiefungen aufweisen, die einen parabel- oder kegelförmigen Querschnitt aufweisen. Solche konkaven Vertiefungen können an jeder Position gebildet werden, an der ein Laserstrahl punktförmig eingestrahlt wurde. Bevorzugt weisen solche konkaven Vertiefungen eine Querschnittsöffnung und eine Tiefe von wenigen Mikrometern, z.B. 1 bis 5 µm auf. Bevorzugt weist der Boden jeder Ausnehmung bzw. jedes Reaktionsgefäßes zumindest 3 konkave Vertiefungen auf.

In Ausführungen, in denen die zweite Oberfläche der ersten Glasplatte vollflächig mit Ätzresist beschichtet ist, wirkt das Ätzen nur von der ersten Oberfläche ein. Die Beschichtung der zweiten Oberfläche führt zur Ausbildung von Ausnehmungen, die von der ersten Oberfläche zylindrisch oder kegelförmig in Richtung auf die zweite Oberfläche zulaufen und verhindert den Ätzabtrag von der zweiten Oberfläche, so dass sich die Ausnehmungen nur bis in einen Anteil der Dicke der Glasplatte erstrecken. In dieser Ausführungsform kann generell das Ätzen ausschließlich auf die erste Oberfläche der Glasplatte einwirken gelassen werden, bis sich die Ausnehmung in die Ebene der zweiten Oberfläche der Glasplatte erstreckt. Es hat sich gezeigt, dass bei Beschichtung der zweiten Oberfläche mit Ätzresist das Ätzen Ausnehmungen in einem Anteil der Dicke der Glasplatte erzeugt, deren Wandung senkrecht bzw. im Winkel der Kegelform bzw. Kegelstumpfform zur Ebene der zweiten Oberfläche steht.

Generell erstrecken sich die Ausnehmungen senkrecht zur ersten Oberfläche in die Glasplatte, die für jede der Ausnehmungen gegenüber der ersten Oberfläche einen Dickenabschnitt als einen einstückigen Boden ausbildet.

Die Reaktionsgefäße weisen z.B. eine Tiefe von zumindest 40 µm, zumindest 50 µm oder zumindest 100 µm oder zumindest 150 µm auf, z.B. bis 250 µm oder bis 200 µm. Die Reaktionsgefäße weisen z.B. einen Durchmesser von zumindest 10 µm oder zumindest 30 µm auf, z.B. bis 200 µm oder bis 1 mm, generell bevorzugt mit eine Streckungsverhältnis von Tiefe zu Durchmesser von zumindest 2, zumindest 4, zumindest 5 oder zumindest 6 auf. Die Ausnehmungen der ersten Glasplatte haben z.B. ein Innenvolumen innerhalb der ersten Glasplatte von 1 pL bis 1 µL.

Die in der Glasplatte gebildeten Ausnehmungen können in einem an die erste Oberfläche angrenzenden oberen Dickenabschnitt 20 einen größeren Querschnitt aufweisen, als in einem daran angrenzenden unteren Dickenabschnitt 21, der in zumindest zwei Teilausnehmungen unterteilt ist. Zwischen den Teilausnehmungen, die sich über den unteren Dickenabschnitt 21 erstrecken, sind optional einstückig aus der Glasplatte über den unteren Dickenabschnitt 21 gebildete Teilwände 22 angeordnet. Diese Teilwände 22 sind voneinander um die Teilausnehmungen beabstandet. Diese Teilwände 22 werden durch Einstrahlen von Laserpulsen, die im Bereich der Teilwände 22 nur maximal in den oberen Dickenabschnitt 20 der Glasplatte eindringen, und Einstrahlen von Laserpulsen im Bereich der Teilausnehmungen, die tiefer in die Glasplatte eindringen, und anschließendes Ätzen der Glasplatte erzeugt. Beim Ätzen bildet sich die im oberen Dickenabschnitt 20, der an die erste Oberfläche 4 der Glasplatte angrenzt, eine Ausnehmung, 2 die sich über den Bereich von zumindest zwei Teilausnehmungen 2' erstreckt. Die Längsmittelachsen 7 der Teilausnehmungen 2' können z.B. in einem Abstand von 10 bis 100 µm angeordnet sein. Die Teilwände 22 erstrecken sich über den unteren Dickenabschnitt zwischen den Teilausnehmungen 2', wobei die Teilausnehmungen 2' in der Ebene der zweiten Oberfläche der Glasplatte 1 z.B. einen Durchmesser von 1 bis 50 aufweisen können. Der obere Dickenabschnitt 20 wird vorliegend auch als erster Dickenabschnitt bezeichnet, der untere Dickenabschnitt 21 wird auch als zweiter Dickenabschnitt bezeichnet. Der obere Dickenabschnitt 20 und der untere Dickenabschnitt 21 erstrecken sich ausgehend von der ersten Oberfläche 4 der Glasplatte 1, unabhängig voneinander z.B. bis in zumindest 20 % oder zumindest 30% oder zumindest 40 oder zumindest 50 %, z.B. bis zu 80 % oder bis zu 70 % oder bis zu 60% der Dicke der ursprünglichen Glasplatte 1. Dabei erstreckt sich der untere Dickenabschnitt 21 in kleinerem oder in größerem Ausmaß in die Dicke der Glasplatte 1 als der obere Dickenabschnitt 20. Generell erstrecken sich die Dickenabschnitte 20, 21 nicht über die ganze Dicke der Glasplatte 1. Z.B. können der erste 20 und insbesondere der zweite Dickenabschnitt 21 so vorbestimmt sein, dass nach dem Ätzen im Bereich der Ausnehmung eine Dicke der Glasplatte 1, bzw. zwischen der Ausnehmung 2 und der zweiten Oberfläche 5, von zumindest 5%, zumindest 10 % oder zumindest 15 % oder zumindest 20 % bestehen bleibt.

Weiter betrifft die Erfindung ein Verfahren zur Analyse, bei dem die Reaktionsgefäße in Glas mit Licht bestrahlt werden und von den Reaktionsgefäßen ausgehendes Licht detektiert wird, sowie die Verwendung der Reaktionsgefäße aus Glas in dem Analyseverfahren.

Dabei kann Licht zur Analyse bevorzugt etwa senkrecht auf die Glasplatte eingestrahlt werden, auf die erste Oberfläche der Glasplatte und/oder in die offenen Ausnehmungen in der Glasplatte, oder auf zweite die Oberfläche der Glasplatte, die ihrer ersten Oberfläche gegenüberliegt.

Es hat sich gezeigt, dass Ausnehmungen, insbesondere Ausnehmungen, die sich kegelförmig von der ersten Oberfläche in das Volumen der Glasplatte erstrecken, bei Bestrahlung mit z.B. sichtbarem Licht mit ihrer Wandung einen deutlichen Kontrast zum Boden der Ausnehmungen bilden. Bei optischer Detektion kann daher der Umfang der Ausnehmungen aufgenommen und dargestellt werden, insbesondere als dunkler Ring im Kontrast zum Boden der Ausnehmungen.

Weiter betrifft die Erfindung Verfahren zur Analyse mit dem Schritt des Bereitstellens von Reaktionsgefäßen, die nach einem erfindungsgemäßen Herstellungsverfahren hergestellt sind, oder die erfindungsgemäße Reaktionsgefäße sind, dem Einbringen von Probe, z.B. Patientenprobe, die zellfrei, z.B. Blutplasma, oder zellhaltig, z.B. Vollblut oder aus Vollblut abgetrennte Zellen, oder Gewebematerial sein kann, in Reaktionsgefäße, vorher, gleichzeitig oder anschließend, Zugeben zumindest eines Reagenzes in Reaktionsgefäße, und Analysieren. Die mit dem Verfahren hergestellten Reaktionsgefäße und die erfindungsgemäßen Reaktionsgefäße haben den Vorteil, dass die Böden der Reaktionsgefäße konkave Vertiefungen aufweisen und Zellen vereinzelt in konkaven Vertiefungen angeordnet sein können.

Optional kann das zumindest eine Reagenz, einfach oder nacheinander mehrfach, in unterschiedlichen Mengen in mehrere Reaktionsgefäße zugegeben werden. Das Reagenz kann z.B. ein pharmazeutischer Wirkstoff sein, und das Analysieren kann die Messung der Wirkung des Wirkstoffs auf die Probe umfassen. Optional kann das Verfahren einen oder mehrere Inkubationsschritte umfassen, z.B. unter Zellkulturbedingungen (37°C, 5% CO₂ Atmosphäre, ruhend oder mit Bewegung).

Das Analysieren kann eine optische Messung der Reaktionsgefäße sein, z.B. während oder nach Sequenzierung von DNA und/oder RNA und/oder von Protein in den Reaktionsgefäßen nach Zugabe von Reagenzien zur Sequenzierung, optional nach Lyse von Zellen, oder die Bestimmung von Proteinen, z.B. nach Reaktion mit einem als Reagenz zugegebenen Bindemolekül, z.B. einem Antikörper, der bevorzugt mit einem Farbstoff markiert ist. Bevorzugt umfasst das Analysieren z.B. bei zellhaltigen Proben die Bestimmung der transkribierten RNAs und/oder der translatierten Proteine, insbesondere für Proben, denen kein Wirkstoff zugegeben wurde im Vergleich mit Proben, denen Wirkstoff zugegeben wurde. Optional kann das Verfahren zur Analyse die Entnahme eines Anteils der Probe aus einem Reaktionsgefäß umfassen, weiter optional das Einfüllen des entnommenen Probeanteils in ein weiteres erfindungsgemäßes oder erfindungsgemäß hergestelltes Reaktionsgefäß. Das Zugeben von Probe und/oder von Reagenz in die Reaktionsgefäße kann z.B. durch Bewegen von Flüssigkeitstropfen der Probe und/oder von Flüssigkeitstropfen des Reagenzes erfolgen, wobei die Flüssigkeitstropfen z.B. als Teil eines Flüssigkeitsstrahls erzeugt und bewegt werden, wie dies z.B. durch Einwirken elektromagnetischer Strahlung, durch Beaufschlagung mit Schall oder Ultraschall, durch Anlegen eines elektrischen Felds oder durch Druckbeaufschlagung erfolgen. Das Erzeugen von Flüssigkeitstropfen ist als Tintenstrahldruckverfahren oder Pipettieren bekannt. Alternativ können laserbasierte Druckverfahren, z.B. Laser-Transfer-Druck, eingesetzt werden. Bevorzugt erfolgt das Zugeben von Probe und/oder von Reagenz ohne Kontakt der Dosiervorrichtung mit dem Reaktionsgefäß.

In Ausführungsformen, in denen Leiterbahnen an den Ausnehmungen angeordnet sind, kann bei Verfahren zur Analyse das Innenvolumen der Ausnehmungen mit Spannung beaufschlagt werden und zwischen den Leiterbahnen können elektrische Parameter gemessen werden, die im Innenvolumen vorliegen.

Die Figuren zeigen schematisch in
- Fig. 1 im Querschnitt senkrecht zur Oberfläche der ersten Glasplatte eine Ausführungsform der Reaktionsgefäße in einer einstückigen Glasplatte,
- Fig. 2 in Aufsicht auf die erste Oberfläche der Glasplatte die optische Analyse eines erfindungsgemäß hergestellten Reaktionsgefäßes, und in
- Fig. 3 a), b), c) weitere Ausführungsformen im Querschnitt senkrecht zur Oberfläche der ersten Glasplatte zeigen.

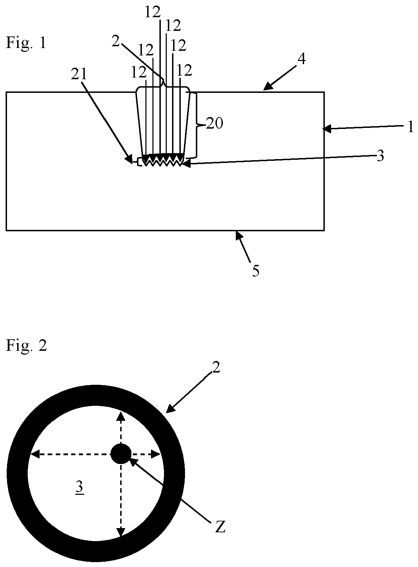

Die Fig. 1 zeigt in einer ersten Glasplatte 1 eine Ausnehmung 2, die durch Ätzen einer ersten Glasplatte 1 nach Einstrahlung eines gepulsten Laserstrahls herstellbar ist. Dabei zeigt der Boden 3 konkave Vertiefungen, die einen etwa parabelförmigen Querschnitt aufweisen. Solche konkaven Vertiefungen werden an jeder Position 12 gebildet, an der ein Laserstrahl punktförmig eingestrahlt wurde. Vorliegend ergibt sich ein Boden 3 mit konkaven Vertiefungen durch punktförmiges Einstrahlen von Laserstrahlen an Positionen in Abständen, die dem Abstand der Mittelpunkte der Vertiefungen entsprechen. Durch das Ätzen, das im Anschluß an das Bestrahlen erfolgt, werden die Bereiche der ersten Glasplatte 1 zwischen den Positionen entfernt. Für diese einstückige Ausführungsform kann der auf die erste Oberfläche 4 eingestrahlte Laserstrahl eingerichtet sein, nur bis zu einem Anteil in das Volumen der ersten Glasplatte 1 einzudringen, und/oder die der ersten Oberfläche 4 gegenüberliegende zweite Oberfläche 5 kann vollflächig mit Ätzresist beschichtet sein.

Die Fig. 2 zeigt in Aufsicht auf eine Glasplatte 1 eine Ausnehmung 2, deren Wandung einen starken optischen Kontrast zum Boden 3 bildet, so dass die Wandung als umfängliche Begrenzung des Bodens 3 deutlich dargestellt wird. Ein Partikel, z.B. eine biologische Zelle Z, kann bei einer durch den Boden 3 gerichteten Beleuchtung mit gutem Kontrast gegen den Boden 3 gesehen werden, insbesondere bei Markierung der Zelle Z durch einen Farbstoff, z.B. einen Fluoreszenzfarbstoff.

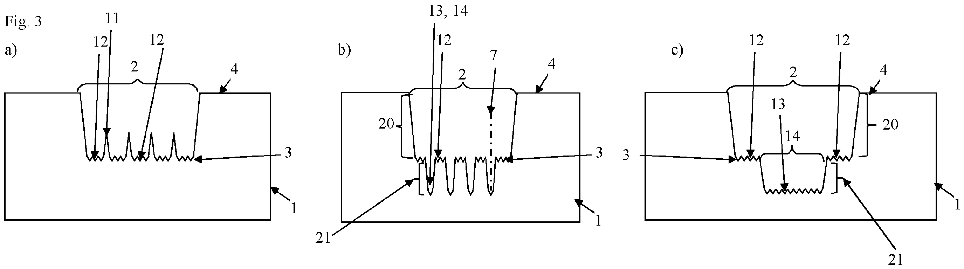

Die Fig. 3 zeigt Ausführungsformen jeweils einer Ausnehmung 2, die einstückig in einer Glasplatte 1 hergestellt sind. Dies zeigt, dass das Einstrahlen von Laserpulsen auf die erste Oberfläche 4 der Glasplatte 1 an mehreren Positionen, die in einem Abstand von z.B. 1 bis 10 µm liegen und daher eine Stelle bilden, an der durch Ätzen genau eine Ausnehmung 2 hergestellt wird. Dabei kann durch das Ätzen an jeder Position 12 am Boden 3 der Ausnehmung 2 jeweils eine konkave Vertiefung als Teilausnehmung 2' erzeugt sein.

Wie in Fig. 3 a) dargestellt, wird eine Glasspitze 11, die senkrecht zur ersten Oberfläche 4 vom Boden 3 in die Ausnehmung 2 ragt, beim Ätzen erzeugt, wenn zumindest 3 Positionen 12, an denen Laserpulse eingestrahlt werden, in größerem Abstand angeordnet sind, z.B. in einem Abstand von 20 µm. Dabei ergibt jede Position 12, an der ein Laserpuls eingestrahlt wurde, beim Ätzen eine konkave Vertiefung bzw. Teilausnehmung 2' im Boden 3.

Die Fig. 3 b) zeigt, dass Laserpulse, die an einzelnen Positionen 13 eingestrahlt werden und tiefer in die Dicke der Glasplatte 1 eindringen, dort beim Ätzen weitere Ausnehmungen 14 gebildet werden, die sich tiefer in die Glasplatte 1 erstrecken, als die Vertiefung an anderen Positionen 12, in denen Laserpulse bis in eine geringere Tiefe in die Glasplatte 1 eingestrahlt wurden. Die Tiefe des Eindringens der Laserpulse in die Glasplatte 1 kann durch Einstellen der Fokuslage und/oder die Stärke der Pulsenergie der Laserpulse vorbestimmt werden.

Die Fig. 3 c) zeigt, dass das Einstrahlen von Laserpulsen, die tiefer in die Glasplatte 1 eindringen, an nebeneinander angeordneten Positionen 13 dort beim Ätzen eine weitere Ausnehmung 14 bilden, die sich tiefer in die Dicke der Glasplatte 1 erstreckt, als die Ausnehmung, deren Boden 3 beim Ätzen von anderen Positionen 12 gebildet wird, an denen die Laserpulse weniger tief in die Glasplatte 1 eingestrahlt worden sind.

### Bezugszeichenliste:

| | | | |
|---|---|---|---|
| 1 | Glasplatte | 11 | Glasspitze |
| 2 | Ausnehmung | 12 | Position eingestrahlter Laserpuls |
| 2' | Teilausnehmung | 13 | Position tiefer eingestrahlter Laserpuls |
| 3 | Boden | 14 | weitere Ausnehmung |
| 4 | erste Oberfläche | 20 | oberer, erster Dickenabschnitt |
| 5 | zweite Oberfläche | 21 | unterer, zweiter Dickenabschnitt |
| 7 | Längsmittelachse | Z | Zelle |
| 8 | Ätzresist | | |

## Patentansprüche

1. Verfahren zur Herstellung einer Vielzahl von Reaktionsgefäßen aus Glas, die als Ausnehmungen (2) in einer einstückigen ersten Glasplatte (1) gebildet sind, mit den Schritten
1. Einstrahlen von Laserpulsen mit Pulslängen von maximal 100 ps an beabstandeten Positionen (12) einer Wellenlänge, für die die Glasplatte (1) durchlässig ist, auf jeweils die Stellen der Glasplatte (1), an denen jeweils eine Ausnehmung (2) als Reaktionsgefäß erzeugt werden soll, wobei der Laser eingerichtet ist, dass der Laserstrahl zwischen den Stellen nicht auf die Glasplatte trifft, wobei die Fokuslage der Laserpulse so eingestellt ist, dass sich der Fokus die Laserpulse nicht über die gesamte Dicke der ersten Glasplatte erstreckt (1),
2. Ätzen der Glasplatte (1), für eine Zeitdauer, die zum Erzeugen von Ausnehmungen (2) mit einer Tiefe entlang der Stellen ausreicht, um die Ausnehmungen (2) zu erzeugen,
3. wobei das Ätzen der Glasplatte (1) beendet wird, wenn die Tiefe der Ausnehmungen (2) nur einen Anteil der Dicke der ersten Glasplatte (2) beträgt und daher die Ausnehmungen (2) einen einstückig in der ersten Glasplatte (1) ausgebildeten Boden (3) aufweisen, wobei an jeder Position (12) einer Stelle eine konkave Vertiefung im Boden gebildet wird.

2. Verfahren nach Anspruch 1, **dadurch gekennzeichnet, dass** die Tiefe der Ausnehmungen (2) mindestens 30 µm beträgt.

3. Verfahren nach einem der voranstehenden Ansprüche, **dadurch gekennzeichnet, dass** die Ausnehmungen (2) ein Streckungsverhältnis von zumindest 2 von Tiefe zu Durchmesser, der in der Ebene der ersten Oberfläche (4) gemessen ist, aufweisen.

4. Verfahren nach einem der voranstehenden Ansprüche, **dadurch gekennzeichnet, dass** die erste Glasplatte (1) an den Stellen, an denen eine Ausnehmung (2) erzeugt werden soll, an mehreren, im gleichen Abstand voneinander beabstandeten Positionen (12) mit Laserpulsen bestrahlt wird.

5. Verfahren nach einem der voranstehenden Ansprüche, **dadurch gekennzeichnet, dass** an einem Teil der Positionen (12) Laserpulse weniger tief bis in einen ersten Dickenabschnitt (20) in die erste Glasplatte (1) eingestrahlt werden, indem die Fokuslage der Laserpulse so eingestellt wird, dass sich der Fokus der Laserpulse nur bis in den ersten Dickenabschnitt (20) erstreckt, und an einem Teil der Positionen (12) Laserpulse tiefer bis in einen angrenzenden zweiten Dickenabschnitt (21) in die erste Glasplatte (1) eingestrahlt werden, indem die Fokuslage der Laserpulse so eingestellt wird, dass sich der Fokus der Laserpulse nur bis in den zweiten Dickenabschnitt (21) erstreckt, wobei beim Ätzen eine Ausnehmung (2), die sich über den ersten Dickenabschnitt (20) erstreckt, gebildet wird und von Teilwänden (22) beabstandete Teilausnehmungen (2'), die sich über den zweiten Dickenabschnitt (21) erstrecken, gebildet werden.

6. Verfahren nach einem der voranstehenden Ansprüche, **dadurch gekennzeichnet, dass** die Positionen (12) in einem Abstand von maximal 10 µm angeordnet sind, wobei zumindest drei Positionen zumindest 20 µm voneinander beabstandet sind.

7. Verfahren nach einem der voranstehenden Ansprüche, **dadurch gekennzeichnet, dass** die Laserpulse mit einer Intensität auf die Glasplatte eingestrahlt werden, dass sie die Glasplatte (1) bis in die Fokuslage modifizieren.

8. Verfahren nach einem der voranstehenden Ansprüche, **dadurch gekennzeichnet, dass** an den Stellen der ersten Glasplatte (1), an denen eine Ausnehmung (2) erzeugt werden soll, ein Laserstrahl auf einem umfänglich geschlossenen Weg eingestrahlt wird.

9. Verfahren nach Anspruch 8, **dadurch gekennzeichnet, dass** der Laserstrahl, der auf einem umfänglich geschlossenen Weg eingestrahlt wird, durch nebeneinander eingestrahlte Laserstrahlpulse gebildet wird.

10. Verfahren nach einem der voranstehenden Ansprüche, **dadurch gekennzeichnet, dass** mindestens eine Seite der ersten Glasplatte (1) vor Schritt 2 mit einem Ätzresist (10) beschichtet wird.

11. Verfahren nach Anspruch 10, **dadurch gekennzeichnet, dass** das Ätzen erfolgt, bis sich angrenzend an die mit Ätzresist (10) beschichtete Oberfläche eine um die Ausnehmung umlaufende Fase (9) bildet.

12. Verfahren nach einem der voranstehenden Ansprüche, **dadurch gekennzeichnet, dass** Leiterbahnen auf die Glasplatte (1) aufgebracht werden, die zumindest einen Anteil der inneren Wandung der Ausnehmungen (2) überdecken.

13. Reaktionsgefäße, die als Ausnehmungen aus Glas gebildet sind, insbesondere erhältlich nach einem Verfahren nach einem der voranstehenden Ansprüche, mit einer Tiefe der Ausnehmungen von zumindest 30 µm in einer Glasplatte (1) und einem Streckungsverhältnis von zumindest 2 von Tiefe zu Durchmesser, der in der Ebene einer ersten Oberfläche (4) der Glasplatte (1) gemessen ist, wobei die Ausnehmungen (2) von Wandungen eingefasst sind, deren Stirnflächen in einer gemeinsamen Ebene angeordnet sind und die erste Oberfläche (4) der ersten Glasplatte (1) bilden, von der die Ausnehmungen (2) ausgenommen sind, **dadurch gekennzeichnet, dass** sich die Ausnehmungen (2) nicht über die ganze Dicke der Glasplatte (1) erstrecken und die Böden (3) der Ausnehmungen von Material der Glasplatte (1) gebildet sind und im Boden zumindest einer Ausnehmung zumindest jeweils drei konkave Vertiefungen ausgebildet sind.

14. Reaktionsgefäße nach Anspruch 13, **dadurch gekennzeichnet, dass** die Böden (3) der Reaktionsgefäße von aneinander angrenzenden Ausnehmungen (2) gebildet sind, die nebeneinander in einer Ebene angeordnet sind, die parallel zur ersten Oberfläche (4) und parallel zur Ebene der dieser gegenüberliegenden zweiten Oberfläche (5) liegt.

15. Reaktionsgefäße nach einem der Ansprüche 13 bis 14, **dadurch gekennzeichnet, dass** in den Böden (3) der Ausnehmungen (2) mindestens eine weitere Ausnehmung (14) gebildet ist, die sich in eine größere Tiefe in der ersten Glasplatte (1) erstreckt.

16. Reaktionsgefäße nach einem der Ansprüche 13 bis 15, **dadurch gekennzeichnet, dass** die Böden (3) zumindest eine einstückig aus der ersten Glasplatte (1) gebildete Glasspitze zur Verwendung als Lichtwellenleiter für eine Nahfeldbeleuchtung der Ausnehmung (2) aufweisen, die sich senkrecht zur ersten Oberfläche (4) der ersten Glasplatte (1) in die Ausnehmung (2) erstreckt.

17. Verfahren zur Analyse einer Probe mit dem Schritt des Bereitstellens von Reaktionsgefäßen, die nach einem Verfahren nach einem der Ansprüche 1 bis 12 hergestellt sind oder die Reaktionsgefäße nach einem der Ansprüche 13 bis 16 sind, mit dem Einbringen von Probe, die zellfrei oder zellhaltig ist, in die Reaktionsgefäße, Zugeben zumindest eines Reagenzes in Reaktionsgefäße, und dem optischen Messen der Reaktionsgefäße.

18. Verfahren nach Anspruch 17, **dadurch gekennzeichnet, dass** die Probe zellhaltig ist und Zellen vereinzelt in konkaven Vertiefungen angeordnet sind.

## Claims

1. Method of production of a plurality of glass reaction vessels formed as recesses (2) in a single-piece first glass plate (1), comprising the steps of
1. irradiation of laser pulses with pulse lengths of at most 100 ps at spaced positions (12) of a wavelength, for which the glass plate (1) is transparent, onto the respective locations of the glass plate (1) at which a respective recess (2) is to be produced as a reaction vessel, wherein the laser is set up such that the laser beam does not strike the glass plate between the locations, wherein the focal position of the laser pulses is adjusted such that the focus of the laser pulses does not extend over the entire thickness of the first glass plate (1),
2. etching the glass plate (1), for a period of time sufficient to produce recesses (2) having a depth along the locations to produce the recesses (2),
3. wherein the etching of the glass plate (1) is terminated when the depth of the recesses (2) is only a proportion of the thickness of the first glass plate (2) and therefore the recesses (2) have a bottom (3) formed single-piece in the first glass plate (1), wherein a concave depression is formed in the bottom at each position (12) of a location.

2. Method according to claim 1, **characterised in that** the depth of the recesses (2) is at least 30 µm.

3. Method according to one of the preceding claims, **characterised in that** the recesses (2) have an aspect ratio of at least 2 of depth to diameter measured in the plane of the first surface (4).

4. Method according to one of the preceding claims, **characterised in that** the first glass plate (1) is irradiated with laser pulses at the locations at which a recess (2) is to be produced, at a plurality of positions (12) spaced at the same distance from one another.

5. Method according to one of the preceding claims, **characterised in that** at some of the positions (12) laser pulses are irradiated less deeply as far as a first thickness section (20) into the first glass plate (1), by adjusting the focus position of the laser pulses such that the focus of the laser pulses extends only into the first thickness section (20), and at some of the positions (12) laser pulses are irradiated more deeply into an adjacent second thickness section (2)1 in the first glass plate (1), by adjusting the focus position of the laser pulses such that the focus of the laser pulses extends only into the second thickness section (21), wherein during etching a recess (2) extending over the first thickness section (20) is formed and partial recesses (2') which are spaced apart by partial walls (22) and which extend over the second thickness section (21) being formed.

6. Method according to one of the preceding claims, **characterised in that** the positions (12) are arranged at a maximum distance of 10 µm, at least three positions being spaced at least 20 µm apart.

7. Method according to one of the preceding claims, **characterised in that** the laser pulses are irradiated onto the glass plate with an intensity such that they modify the glass plate (1) up to the focal position.

8. Method according to one of the preceding claims, **characterised in that** a laser beam is irradiated along a circumferentially closed path at the locations of the first glass plate (1) at which a recess (2) is to be produced.

9. Method according to claim 8, **characterised in that** the laser beam, which is irradiated on a circumferentially closed path, is formed by laser beam pulses irradiated side by side.

10. Method according to one of the preceding claims, **characterised in that** at least one side of the first glass plate (1) is coated with an etching resist (10) prior to step 2.

11. Method according to claim 10, **characterised in that** the etching is carried out until a chamfer (9) forms around the recess adjacent to the surface coated with etching resist (10).

12. Method according to one of the preceding claims, **characterised in that** step conductors are applied to the glass plate (1) which cover at least a portion of the inner wall of the recesses (2).

13. Reaction vessels, which are formed as recesses of glass, in particular obtainable by a method according to one of the preceding claims, with a depth of the recesses of at least 30 µm in a glass plate (1) and an aspect ratio of at least 2 of depth to diameter, which is measured in the plane of a first surface (4) of the glass plate (1), the recesses (2) being enclosed by walls, of which the end faces are arranged in a common plane and form the first surface (4) of the first glass plate (1), from which the recesses (2) are worked off, **characterised in that** the recesses (2) do not extend over the entire thickness of the glass plate (1) and the bottoms (3) of the recesses are formed by material of the glass plate (1) and at least three concave depressions are formed in the bottom of at least one recess in each case.

14. Reaction vessels according to claim 13, **characterised in that** the bottoms (3) of the reaction vessels are formed by adjacent recesses (2) which are arranged next to one another in a plane which is parallel to the first surface (4) and parallel to the plane of the second surface (5) opposite thereto.

15. Reaction vessels according to one of claims 13 to 14, **characterised in that** at least one further recess (14) is formed in the bottoms (3) of the recesses (2), and extends to a greater depth into the first glass plate (1).

16. Reaction vessels according to one of claims 13 to 15, **characterised in that** the bottoms (3) have at least one glass tip formed in a single piece from the first glass plate (1) for use as an optical waveguide for near-field illumination of the recess (2), which tip extends into the recess (2) perpendicularly to the first surface (4) of the first glass plate (1).

17. Method of analysing a sample comprising the step of providing reaction vessels which are produced by a method according to any one of claims 1 to 12 or which are reaction vessels according to any one of claims 13 to 16, introducing a sample, which is cellfree or cell-containing, into the reaction vessels, adding at least one reagent into reaction vessels, and optically measuring the reaction vessels.

18. Method according to claim 17, **characterised in that** the sample contains cells and cells are arranged singly in concave depressions.

## Revendications

1. Procédé de fabrication d'une pluralité de récipients de réaction en verre, qui sont formés comme des évidements (2) dans une première plaque de verre (1) d'un seul tenant, comprenant les étapes suivantes
1. l'irradiation d'impulsions laser avec des longueurs d'impulsion de 100 ps au maximum à des positions espacées (12) d'une longueur d'onde pour laquelle la plaque de verre (1) est transparente, respectivement sur les endroits de la plaque de verre (1) où un évidement (2) doit respectivement être produit comme récipient de réaction, le laser étant aménagé de telle sorte que le rayon laser ne rencontre pas la plaque de verre entre les endroits, la position focale des impulsions laser étant réglée de telle sorte que le foyer des impulsions laser ne s'étend pas sur toute l'épaisseur de la première plaque de verre (1),
2. gravure de la plaque de verre (1), pendant une période de temps suffisante pour produire des évidements (2) ayant une profondeur le long des endroits pour produire les évidements (2),
3. dans lequel la gravure de la plaque de verre (1) est terminée lorsque la profondeur des évidements (2) est seulement une proportion de l'épaisseur de la première plaque de verre (2) et, par conséquent, les évidements (2) ont un fond (3) formé d'un seul tenant dans la première plaque de verre (1), dans lequel un creux concave est formé dans le fond à chaque position (12) d'un endroit.

2. Procédé selon la revendication 1, **caractérisé en ce que** la profondeur des évidements (2) est d'au moins 30 µm.

3. Procédé selon l'une quelconque des revendications précédentes, **caractérisé en ce que** les évidements (2) ont un rapport d'étirement d'au moins 2 de la profondeur au diamètre, mesuré dans le plan de la première surface (4).

4. Procédé selon l'une des revendications précédentes, **caractérisé en ce que** la première plaque de verre (1) est irradiée par des impulsions laser aux endroits où un évidement (2) doit être créé, en plusieurs positions (12) espacées d'une même distance.

5. Procédé selon l'une des revendications précédentes, **caractérisé en ce que**, dans une partie des positions (12), des impulsions laser sont irradiées moins profondément jusque dans une première section d'épaisseur (20) dans la première plaque de verre (1), en réglant la position focale des impulsions laser de telle sorte que le foyer des impulsions laser ne s'étende que jusque dans la première section d'épaisseur (20), et **en ce que**, dans une partie des positions (12), des impulsions laser sont irradiées plus profondément jusque dans une deuxième section d'épaisseur (21) adjacente dans la première plaque de verre (1), en réglant la position focale des impulsions laser de telle sorte que le foyer des impulsions laser ne s'étende que jusque dans la deuxième section d'épaisseur (21), un évidement (2) s'étendant sur la première section d'épaisseur (20) étant formé lors de la gravure et des évidements partiels (2') espacés par des parois partielles (22) et s'étendant sur la deuxième section d'épaisseur (21) étant formés.

6. Procédé selon l'une des revendications précédentes, **caractérisé en ce que** les positions (12) sont disposées à une distance maximale de 10 µm, au moins trois positions étant espacées d'au moins 20 µm.

7. Procédé selon l'une des revendications précédentes, **caractérisé en ce que** les impulsions laser sont irradiées sur la plaque de verre avec une intensité telle qu'elles modifient la plaque de verre (1) jusqu'à la position focale.

8. Procédé selon l'une quelconque des revendications précédentes, **caractérisé en ce que**, aux endroits de la première plaque de verre (1) où un évidement (2) doit être créé, un faisceau laser est irradié selon un trajet fermé sur sa circonférence.

9. Procédé selon la revendication 8, **caractérisé en ce que** le faisceau laser, qui est irradié sur un trajet fermé sur sa circonférence, est formé par des impulsions de faisceau laser irradiées côte à côte.

10. Procédé selon l'une quelconque des revendications précédentes, **caractérisé en ce qu'**au moins une face de la première plaque de verre (1) est revêtue d'une réserve de gravure (10) avant l'étape 2.

11. Procédé selon la revendication 10, **caractérisé en ce que** la gravure est effectuée jusqu'à ce qu'un chanfrein (9) entourant l'évidement se forme de manière adjacente à la surface revêtue de réserve de gravure (10).

12. Procédé selon l'une des revendications précédentes, **caractérisé en ce que** des pistes conductrices sont appliquées sur la plaque de verre (1), qui recouvrent au moins une partie de la paroi intérieure des évidements (2).

13. Récipients de réaction qui sont formés comme des évidements en verre, pouvant être obtenus en particulier selon un procédé selon l'une des revendications précédentes, avec une profondeur des évidements d'au moins 30 µm dans une plaque de verre (1) et un rapport d'étirement d'au moins 2 de la profondeur au diamètre, qui est mesuré dans le plan d'une première surface (4) de la plaque de verre (1), les évidements (2) étant entourés par des parois, dont les faces frontales sont disposées dans un plan commun et forment la première surface (4) de la première plaque de verre (1), de laquelle les évidements (2) sont exclus, **caractérisé en ce que** les évidements (2) ne s'étendent pas sur toute l'épaisseur de la plaque de verre (1) et les fonds (3) des évidements sont formés par du matériau de la plaque de verre (1) et au moins trois évidements concaves sont formés dans le fond d'au moins un évidement.

14. Récipients de réaction selon la revendication 13, **caractérisés en ce que** les fonds (3) des récipients de réaction sont formés par des évidements (2) adjacents les uns aux autres, qui sont disposés les uns à côté des autres dans un plan qui est parallèle à la première surface (4) et parallèle au plan de la deuxième surface (5) opposée à celle-ci.

15. Récipients de réaction selon l'une quelconque des revendications 13 à 14, **caractérisés en ce qu'**au moins un autre évidement (14) est formé dans les fonds (3) des évidements (2), lequel s'étend à une profondeur plus importante dans la première plaque de verre (1).

16. Récipients de réaction selon l'une quelconque des revendications 13 à 15, **caractérisés en ce que** les fonds (3) comportent au moins une pointe de verre formée d'un seul tenant à partir de la première plaque de verre (1) et destinée à être utilisée comme guide d'ondes lumineuses pour un éclairage en champ proche de l'évidement (2), qui s'étend dans l'évidement (2) perpendiculairement à la première surface (4) de la première plaque de verre (1).

17. Procédé d'analyse d'un échantillon comprenant l'étape consistant à fournir des récipients de réaction qui sont préparés selon un procédé selon l'une quelconque des revendications 1 à 12 ou qui sont des récipients de réaction selon l'une quelconque des revendications 13 à 16, comprenant l'introduction d'un échantillon qui est exempt de cellules ou qui contient des cellules dans les récipients de réaction, l'addition d'au moins un réactif dans des récipients de réaction, et la mesure optique des récipients de réaction.

18. Procédé selon la revendication 17, **caractérisé en ce que** l'échantillon contient des cellules et que des cellules sont disposées de manière isolée dans des évidements concaves.
